# EUROPEAN PATENT APPLICATION

(11) **EP 4 083 626 A1**
(43) Date of publication of application: **02.11.2022**
(21) Application number: 20908194.2
(22) Date of filing: 11.09.2020
(51) Int. Cl.: G01N 33/543, G01N 33/558, G01N 33/52, B01L 3/00

(54) **METHOD FOR MANUFACTURING TEST STRIP FOR MULTIPLEX IMMUNOASSAY ANALYSIS, AND TEST STRIP MANUFACTURED USING SAME**

(30) Priority: 26.12.2019 KR 20190175150
(71) Applicant: Precision Biosensor Inc., Daejeon 34036 (KR)
(72) Inventor: HWANG, Kyu Youn, Sejong-si 30064 (KR); PARK, Jong-Myeon, Seongnam-si Gyeonggi-do 13589 (KR)
(74) Representative: Sander, Rolf
(86) International application number: PCT/KR2020/012332
(87) International publication number: WO 2021/132847

(57) **Abstract**

The present disclosure relates to a method for manufacturing a test strip for multiplex immunoassay analysis and the test strip manufactured using same. The present invention relates to a test strip for multiplex immunoassay analysis and a multiplex immunoassay analysis device using same, and provides a method for manufacturing a test strip for multiplex immunoassay analysis and the test strip manufactured using same, which include a first reactant linked to a fluorescent substance and a second reactant linked to a reflective light substance that are applied as reactants for detecting a target analyte in a biosample, thereby enabling both fluorescence detection and reflected light detection of the target analyte in one reaction region so as to improve diagnosis accuracy, and that can be selectively applied to a fluorescent-type immunoanalytical diagnostic device or a reflective-type immunoanalytical diagnostic device according to the needs of a user so as to increase ease of use, and can detect two target analytes through fluorescent light and reflective light in one reaction region so as to enable multiplex analysis by means of a simple and miniaturized structure having one reaction region.

## Description

### TECHNICAL FIELD

The present disclosure generally relates to a method for manufacturing a test strip for multiplex immunoassay analysis and a test strip manufactured using the same, and more particularly, a method for manufacturing a test strip for multiplex immunoassay analysis and a test strip manufactured using the same in which, by using reactants for detection of target analytes in a biopsy sample which are a first reactant to which fluorescent material is coupled and a second reactant to which reflected light material is coupled, both fluorescent detection and reflected light colorimetric detection associated with the target analytes can be performed at one reaction area, thereby improving diagnosis accuracy, a fluorescent type immunoassay analysis diagnosis apparatus or a reflected light colorimetric type immunoassay analysis apparatus can be selectively used depending on the user's necessity, thereby increasing use convenience, two target analytes can be detected at one reaction area using fluorescence and reflected light, and one reaction area is included to provide a simple and minimized structure while being capable of performing multiple analysis.

### BACKGROUND

A rapid diagnostic test kit for immunoassay is a test tool for point-of-care capable of performing diagnostic test using a biopsy sample such as blood, urine, saliva, and the like. Examples of such rapid diagnostic test kits include a pregnancy diagnostic kit, an AIDS diagnostic kit, and the like.

Such a diagnostic device must establish a method capable of detecting a certain biological material (protein, DNA, antigen, etc.) for diagnosis. As a conventional biopsy detection method, there may be a fluorescent labeling method using organic dye and the like, and a reflected light labeling method using reflected light material and the like.

This rapid immunoassay diagnostic apparatus operates on the same operation principle as either the fluorescent type or the reflected light type, and uses a test strip capable of adsorbing and supplying a biopsy sample through a capillary phenomenon. Because the biopsy sample supplied from the outside of the test strip flows laterally in the longitudinal direction in the test strip due to the capillary phenomenon, this method is also referred to as a lateral flow immunoassay.

In general, the test strip includes a sample pad, a block pad, a probe pad, a membrane, and an adsorption pad sequentially disposed in a longitudinal direction. The biopsy sample is supplied to the sample pad from the outside, the supplied biopsy sample sequentially flows to the block pad, the probe pad, the membrane, and the adsorption pad by a capillary phenomenon. A block body is applied to the block pad so that the block body can bind with a target analyte (antigen, etc.) in the biopsy sample, and a reactant for block eluted from the block pad and the target analyte are coupled, thereby forming the biosynthetic for block. A reactant for detection is applied to the probe pad to be coupled to the target analyte, and the target analyte coupled with the reactant for block at the block pad is coupled with a reactant eluted from the probe pad, thereby forming the biosynthetic for detection. A reaction area to which capturing material is applied to capture the biosynthetic for detection is formed at a membrane, and the biosynthetic for detection and the capturing material are coupled at the reaction area, thereby forming the bio-complex for detection. The adsorption pad is made of a porous material to enable adsorption movement of the biopsy sample.

When the test light is radiated to the reaction area of the test strip, fluorescence is generated in the bio-complex for detection in the reaction area or reflected light is generated by reflecting the test light depending on the type of the reactant for detection, and diagnosis related to specific disease factors is performed by detecting the fluorescence or the reflected light.

According to this structure, the fluorescence type immunoassay diagnostic apparatus may use ultraviolet light or a specific excitation wavelength according to the type of fluorescent material as the type of test light, and therefore fluorescence is generated from the bio-complex for detection to detect fluorescence, and the reflected light type immunoassay diagnostic apparatus uses visible light as the type of test light, and therefore the test light is reflected from the bio-complex to detect the reflected light. It is possible to diagnose whether there is a specific disease factor by analyzing the detection intensity of the detected fluorescence or reflected light.

The fluorescence-type immunoassay diagnostic device and reflected light-type immunoassay diagnostic device have not only light sources of test light different from each other, but also compositions of reactants for detection of test strips different from each other, and therefore the testing for their combination is impossible, so research and development have been conducted in independent methods for each to date.

In addition, the test strip is generally formed to have one reaction region, and therefore only one specific disease factor can be tested at one time, but it cannot be tested whether there are a number of specific disease factors, and because it is impossible to determine the accuracy of the corresponding test accuracy, the test must be performed one more time and be verified in order to determine the accuracy of the one-time test.

In order to test a number of specific disease factors through a single test using one test strip or to verify test accuracy, methods of increasing the number of reaction regions formed on the test strip have been proposed, but the length of the test strip is increased, and there were problems such as increase in the amount of a biopsy sample and increase in the reaction time.

Specially, because diseases are linked with complex biological interactions and various proteins are intricately related to diseases, there is growing clinical demand for a diagnostic method that allows multiple analysis at a single test for accurate diagnosis, but a solution for this has not been provided in the conventional immunoassay diagnostic apparatus.

### DETAILED DESCRIPTION OF DISCLOSURE

### Technical Problem

To solve the problem of the conventional art, the purpose of the present disclosure may be for providing a method for manufacturing a test strip for multiplex immunoassay analysis and a test strip manufactured using the same in which, by using reactants for detection of target analytes in a biopsy sample which are a first reactant to which fluorescent material is coupled and a second reactant to which reflected light material is coupled, both fluorescent detection and reflected light detection associated with the target analytes can be performed at one reaction area and the Hook effect which is a chronic problem of the immune response can be resolved, thereby improving diagnosis accuracy as well as providing more accurate and various types of diagnosis without adding an additional reaction area, and therefore performing multiple diagnosis function and making possible of miniaturization and structure simplification.

Another purpose of the present disclosure may be for providing a method for manufacturing a test strip for multiplex immunoassay analysis and a test strip manufactured using the same in which a fluorescent type immunoassay analysis diagnosis apparatus or a reflected light type immunoassay analysis apparatus can be selectively used depending on the user's necessity, thereby increasing use convenience and expanding a usable range.

Still another purpose of the present disclosure may be for providing a method for manufacturing a test strip for multiplex immunoassay analysis and a test strip manufactured using the same in which two target analytes can be detected at one reaction area using fluorescence and reflected light, and only one reaction area is included to provide a simple and minimized structure, thereby being capable of diagnosing two specific disease factors in one time test.

### Solution to Problem

According to an embodiment of the present disclosure, a method of manufacturing a test strip for multiple immunoassay analysis may comprise: a sample pad manufacturing step of manufacturing a sample pad so that a biopsy sample is supplied; a probe pad manufacturing step of manufacturing a probe pad to include a first reactant for detection and a second reactant for detection coupled to target analytes, respectively; a capturing membrane manufacturing step of manufacturing a capturing membrane to form a reaction area able to capture two biosynthetics generated by coupling the first reactant for detection and the second reactant for detection to the target analytes, respectively; an absorption pad manufacturing step of manufacturing an absorption pad for absorption movement of the biopsy sample; and an arrangement and fixation step of sequentially arranging and fixing the sample pad, the probe pad, the capturing membrane and the absorption pad in order of a direction of flow of the biopsy sample, wherein: the first reactant for detection is formed in a structure in which first probe material forming fluorescence by radiation of test light is fixedly coupled to the first reactant for detection, and the second reactant for detection is formed in a structure in which second probe material forming reflected light by the radiation of the test light is fixedly coupled to the second reactant for detection.

At this embodiment, the probe pad manufacturing step may comprise: a first probe solution manufacturing step of manufacturing first probe solution containing the first reactant for detection; a second probe solution manufacturing step of manufacturing second probe solution containing the second reactant for detection; a step in which the first probe solution and the second probe solution are absorbed to a glass fiber substrate; and a step of drying the glass fiber substrate to which the first probe solution and the second probe solution are absorbed.

Additionally, the probe pad manufacturing step may further comprise a step of drying and storing the dried glass fiber substrate in a space at humidity of 20% or less.

Further, the first probe material may be an Eu (europium) particle.

In addition, the first probe solution manufacturing step may comprise: a step of washing the Eu particle having -COOH on a surface of the Eu particle by MES(N-morpholino ethanesulfonic acid) binding buffer; and a step of forming an amin-responsive Eu particle by reacting the washed Eu particle and EDC/Sulfo-NHS((N-(3-dimethylpropyl)-N-ethycarbodiimide hydrochloride)/N-hydroxysulfosuccinimide); and a step in which the amin-responsive Eu particle is mixed and reacted with biopsy material capable of being coupled to at least one of the target analytes, and wherein, in the step in which the amin-responsive Eu particle is mixed and reacted with the biopsy material, the amin-responsive Eu particle is coupled to the biopsy material, thereby forming the first reactant for detection.

Further, the second probe material may be an Au particle.

Additionally, the second reactant for detection may be formed by a way of being coupled by ion interaction between sodium citrate ion coated on a surface of a gold particle and a cationic functional group of biopsy material.

In addition, the capturing membrane manufacturing step may comprise: a step of preparing SA-glutaraldehyde (streptavidin-glutaraldehyde) solution; a step of mixing biopsy material capable of being coupled to the target analytes with the SA-glutaraldehyde to be diluted to SPB (sodium phosphate buffer) to make diluted solution; a step of forming the reaction area by spraying and fixing the diluted solution to a specific area of a nitrocellulose membrane; and a step of blocking the nitrocellulose membrane processed through the step of forming the reaction area in mixed solution including NFDM (non-fat dry milk).

Further, the capturing membrane manufacturing step may further comprise a step of drying and storing the blocked nitrocellulose membrane in a space at humidity of 20% or less.

Additionally, the step of preparing SA-glutaraldehyde may comprise: a step of dissolving SA (streptavidin) in the SPB; a step of adding glutaraldehyde to solution, in which the SA is dissolved, to be mixed and reacted; a step of dialyzing solution, processed through the step of adding the glutaraldehyde to the solution to be mixed and reacted, with the SPB.

In addition, the method of manufacturing the test strip for multiple immunoassay analysis may further comprise a block pad manufacturing step of manufacturing a block pad to include a reactant for block coupled to the target analytes in the biopsy sample, wherein the arrangement and fixation step sequentially arranges and fixes the sample pad, the block pad, the probe pad, the capturing membrane and the absorption pad in order of the direction of flow of the biopsy sample.

Further, the block pad manufacturing step may comprise: a block solution preparation step of preparing block solution including the reactant for block; a step in which the block solution together with mouse immunoglobulin G is absorbed to a glass fiber substrate; and a step of drying the glass fiber substrate to which the block solution is absorbed.

Additionally, the block pad manufacturing step may further comprise a step of drying and storing the dried glass fiber substrate in a space at humidity of 20% or less.

In addition, the block pad manufacturing step may further comprise: a step of dissolving NHS-biotin (N-hydroxysuccinimide-biotin) in DMSO(Dimethyl sulfoxide); a step of adding biopsy material capable of being coupled to at least one of the target analytes to solution, in which the NHS-biotin is dissolved, to be mixed and reacted; and a step of dialyzing solution, processed by the step of adding the biopsy material to the solution in which the NHS-biotin is dissolved to be mixed and reacted, with the SPB, wherein, in the step of adding the biopsy material to the solution in which the NHS-biotin is dissolved to be mixed and reacted, the NHS-biotin is coupled to the biopsy material, thereby forming the reactant for block.

Further, in a case that the first reactant for detection and the second reactant for detection are formed to be coupled to different types of target analytes, respectively, the reactant for block may comprise a first reactant for block capable of being coupled to the first target analyte for detection, and a second reactant for block capable of being coupled to the second target analyte for detection.

According to another embodiment of the present disclosure, a test strip for multiple immunoassay analysis is manufactured by the method of manufacturing the test strip for multiple immunoassay analysis described above.

### Advantageous Effects of Invention

According to the present disclosure, there are technical effects in that, by using reactants for detection of target analytes in a biopsy sample which are a first reactant to which fluorescent material is coupled and a second reactant to which reflected light material is coupled, both fluorescent detection and reflected light detection associated with the target analytes can be performed at one reaction area and the Hook effect which is a chronic problem of the immune response can be resolved, thereby improving diagnosis accuracy as well as providing more accurate and various types of diagnosis without adding an additional reaction area, and therefore performing multiple diagnosis function and making possible of miniaturization and structure simplification.

Additionally, there are technical effects in that a fluorescent type immunoassay analysis diagnosis apparatus or a reflected light type immunoassay analysis apparatus can be selectively used depending on the user's necessity, thereby increasing use convenience and expanding a usable range.

Further, there are technical effects in that two target analytes can be detected at one reaction area using fluorescence and reflected light, and only one reaction area is included to provide a simple and minimized structure, thereby being capable of diagnosing two specific disease factors in one time test.

### BRIEF DESCRIPTION OF THE DRAWINGS

FIG. 1 is a figure illustrating a test scrip for multiplex immunoassay analysis according to an embodiment of the present disclosure.
FIG. 2 is a figure conceptually illustrating a structure of a reactant for detection of a test scrip for multiplex immunoassay analysis according to an embodiment of the present disclosure.
FIGS. 3 and 4 are figures conceptually illustrating structures of biosynthetic captured at a reaction area of a test scrip for multiplex immunoassay analysis according to an embodiment of the present disclosure.
FIG. 5 is a figure of conceptually illustrating structures of reactants for block of a test strip for multiplex immunoassay analysis according to an embodiment of the present disclosure.
FIG. 6 is a figure for conceptually illustrating a structure of biosynthetic captured at a reaction area in a case that a block pad is included in a test strip for multiplex immunoassay analysis according to an embodiment of the present disclosure.
FIG. 7 is a flowchart of a process of manufacturing a test strip for multiplex immunoassay analysis according to an embodiment of the present disclosure.
FIG. 8 is a flowchart of illustrating a process of manufacturing a block pad in more detail in a process of manufacturing a test strip for multiplex immunoassay analysis according to an embodiment of the present disclosure.
FIG. 9 is a flowchart of illustrating a process of manufacturing a probe pad in more detail in a process of manufacturing a test strip for multiplex immunoassay analysis according to an embodiment of the present disclosure.
FIG. 10 is a flowchart of illustrating a process of manufacturing a capturing membrane in more detail in a process of manufacturing a test strip for multiplex immunoassay analysis according to an embodiment of the present disclosure.
FIG. 11 is a graph showing linearity according to densities of a TnI sample and a NT-proBNP sample of a test strip for multiplex immunoassay analysis according to an embodiment of the present disclosure.
FIG. 12 is a graph of showing correlation according to concentrations of a TnI sample and a NT-proBNP sample of an immunoassay test strip according to an embodiment of the present disclosure.
FIG. 13 is a graph of illustrating linear correlation for measuring CRP concentration of a multiple immunoassay test strip according to an embodiment of the present disclosure.

### DESCRIPTION OF EMBODIMENTS OF DISCLOSURE

Hereinafter, preferred embodiments of the present disclosure will be described in detail with reference to the accompanying drawings. Throughout this document, reference should be made to the drawings, in which the same reference numerals and symbols will be used to designate the same or like components. Additionally, in the following description of the present disclosure, detailed descriptions of known components and functions incorporated herein will be omitted in the case that the subject matter of the present disclosure may be rendered unclear thereby.

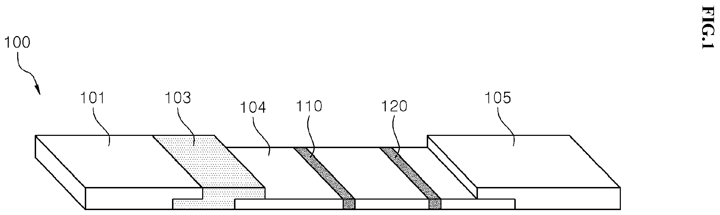

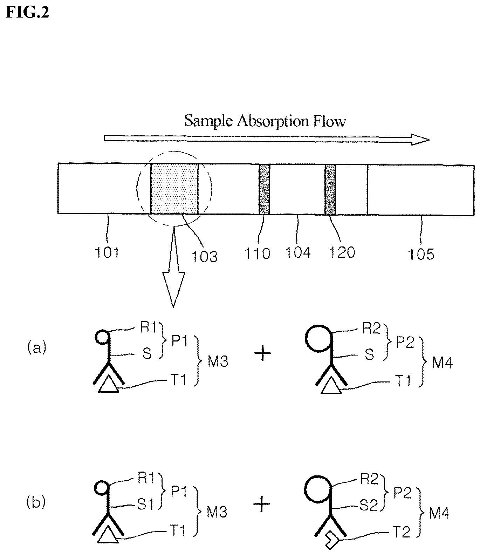

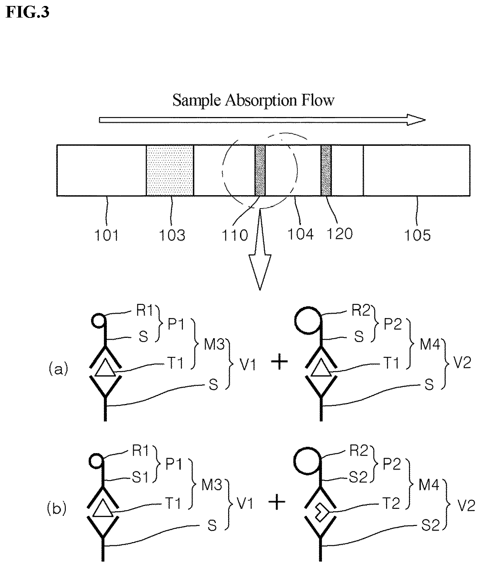

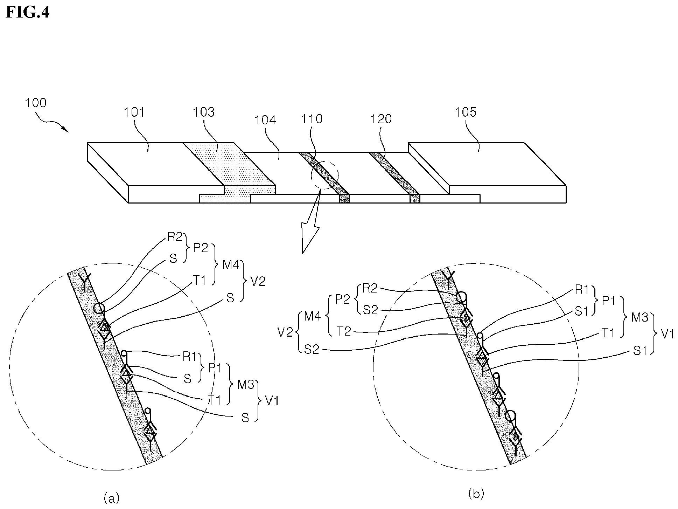

FIG. 1 is a figure illustrating a test scrip for multiplex immunoassay analysis according to an embodiment of the present disclosure, and FIG. 2 is a figure conceptually illustrating a structure of a reactant for detection of a test scrip for multiplex immunoassay analysis according to an embodiment of the present disclosure. And, FIGS. 3 and 4 are figures conceptually illustrating structures of biosynthetic captured at a reaction area of a test scrip for multiplex immunoassay analysis according to an embodiment of the present disclosure.

Referring to FIGS. 1 to 4, a test strip for multiplex immunoassay analysis according to an embodiment of the present disclosure has a structure capable of fluorescence detection as well as reflected light detection, and a sample pad (101), a probe pad (103), a capturing membrane (104) and an absorption pad (105) are sequentially in order along a length direction of the test strip (100).

The configurations of the sample pad (101), the probe pad (103), the capturing membrane (104) and the absorption pad (105) are configured to perform the same as the functions of those of a general test strip described in the Background.

Accordingly, a biopsy sample is supplied to the sample pad (101), and the supplied biopsy sample is sequentially moved in order of the probe pad (103), the capturing membrane (104) and the absorption pad (105) by a capillary phenomenon.

Reactants (PI, P2) for detection are applied to the probe pad (103) to be coupled or bound to target analytes (T1, T2), and the target analytes (T1, T2) and the reactants (P1, P2) for detection are bound or linked to each other at the probe pad (103) and therefore biosynthetic (M3, M4) is formed. A reaction area (110) to which capturing material (biopsy material) (S) is applied to capture the biosynthetic (M3, M4) for detection is formed at the capturing membrane (104), and the biosynthetic (M3, M4) and the capturing material (biopsy material) (S) are bound to each other at the reaction area (110) and then bio-complexes (VI, V2) for detection are formed. The absorption pad (105) is made of porous material so that absorption movement of the biopsy sample can occur.

In short, the target analytes (T1, T2) among the biopsy sample provided to the sample pad (101) are bound or coupled to the reactants (PI, P2) for detection at the probe pad (103) thereby forming the biosynthetic (M3, M4) for detection, and during the process of the continuous movement of the biosynthetic (M3, M4) for detection along the capturing membrane (104) toward the absorption pad (105) the biosynthetic (M3, M4) for detection is captured and bounded by the capturing material (biopsy material)(S) at the reaction area (110) formed at the capturing membrane (104) thereby forming the bio-complexes (VI, V2) for detection.

The reaction area (110) of the test strip (100) is formed in a line shape crossing the capturing membrane (104) in the width direction, and is formed to have one reaction area (110) and one control area (120). If necessary, the plurality of reaction areas (110) may be formed to be separated and spaced apart from each other. Whether a specific disease factor exists is diagnosed by detecting fluorescence or reflected light from the bio-complexes (VI, V2) for detection formed at the reaction area (110), and the validity of the test can be determined through the control area (120).

According to one embodiment of the present disclosure, the reactants (P1, P2) for detection applied to the probe pad (103) comprise a first reactant (P1) for detection and a second reactant (P2) for detection.

A first probe material (R1) configured to form fluorescence by the radiation of test light is fixedly coupled to the first reactant (P1) for detection, and a second probe material (R2) configured to form reflected light by reflecting the test light during the radiation of the test light is fixedly coupled to the second reactant (P2) for detection.

Specifically, the first reactant (P1) for detection and the second reactant (P2) for detection are applied together as the reactants (P1, P2) for detection to the probe pad (103), and these reactants (P1, P2) for detection can be formed in a structure in which the probe material (R1, R2) is fixedly coupled to the biopsy material (S).

In the first reactant (P1) for detection, the first probe material (R1) forming fluorescence during the radiation of the test light (ultraviolet light and excitation light by fluorescent material) is fixedly coupled to the biopsy material (S), and fluorescent dye, quantum dot, lanthanide material (lanthanides: Eu, Tb, Dy, Sm) and so on can be used as the first probe material (R1).

In the second reactant (P2) for detection, the second probe material (R2) forming the reflected light by reflecting test light (visible light) during the radiation of the test light is fixedly coupled to the biopsy material (S), and metal nano particles, for example, gold (Au), silver (Ag), copper (Cu), aluminum (Al), nickel (Ni), cobalt (Co), iron (Fe), zinc (Zn), manganese (Mn) and so on can be used as the second probe material (R2) and in addition carbon particles, latex particles and so on can be used.

These first reactant (P1) for detection and second reactant (P2) for detection can be formed by a gas phase condensation method, a high frequency plasma chemical synthesis method, a conventional chemical precipitation, a hydrothermal synthesis method, an electric dispersion re-action method, a combustive synthesis method, a sol-gel synthesis method, a thermochemical synthesis method, a microfludizer process, a microemulson technology, a high energy mechanical milling or their combination.

As illustrated in FIG. 2, the first reaction (P1) for detection is specifically coupled to the target analyte (T1) which is a disease marker in the biopsy sample, thereby forming the biosynthetic (M3) for fluorescent detection, and the second reaction (P2) for detection is specifically coupled to the target analyte (T1) which is a disease marker in the biopsy sample, thereby forming the biosynthetic (M4) for reflected light detection.

At that time, as illustrated in FIG. 2(a), the first reactant (P1) for detection and the second reactant (P2) for detection are configured to be specifically coupled to the same target analyte (T1), and, as illustrated in FIG. 2(b), the first reactant (P1) for detection and the second reactant (P2) for detection are configured to be specifically coupled to target analytes (T1, T2) different from each other, respectively.

In the exemplary embodiment illustrated in FIG. 2(a), both the first reactant (P1) for detection and the second reactant (P2) for detection are specifically coupled to the same target analyte (T1) in the biopsy sample, thereby forming the biosynthetic (M3) for fluorescent detection and the biosynthetic (M4) for reflected light detection, respectively.

The formed biosynthetic (M3) for fluorescent detection and the formed biosynthetic (M4) for reflected light detection are spread toward the absorption pad (105) in the capturing membrane (104), and, during this process, as illustrated in FIG. 3(a) and FIG. 4(a), are captured by the biopsy material (S) applied to the reaction area (110). Here, the biopsy material (S) applied to the reaction area (110) is material specifically couplable to the target analyte (T1) or other biopsy material in the biopsy sample, and may be, for example, nucleic acid comprising DNA or RNA, amino acid, fat, glycoprotein, antibody, antigen, or their combination. In the reaction area (110), the biosynthetic (M3) for fluorescent detection and the biosynthetic (M4) for reflected light detection are coupled to the biopsy material (S), thereby forming the bio-complex (VI) for fluorescent detection and the bio-complex (V2) for reflected light detection, respectively.

The biosynthetic (M1) for fluorescent detection and the biosynthetic (M2) for reflected light detection are captured thereby forming the bio-complex (VI) for fluorescent detection and the bio-complex (V2) for reflected light detection, and therefore, when the test light (ultraviolet light) is radiated to the reaction area (110), fluorescence is formed by the first probe material (R1) of the bio-complex (VI) for fluorescent detection and the fluorescence can be detected, and likewise, when the test light (visible light) is radiated to the reaction area (110), the reflected light is formed in response to the test light by the second probe material (R2) of the bio-complex (V2) for the reflected light detection, and the reflected light can be detected.

Illumination light with an ultraviolet (UV) wavelength range, for example, illumination light with 400nm or less wavelength, may be used for the test light for fluorescent detection. For this, the UV LED can be used as a source of light and the excitation wavelength of a fluorescent material characteristic can be used. Illumination light of visible light series can be used for the test light for the reflected light detection, and various light sources such as red light LEDs, green light LEDs, blue light LEDs, and so on can be used as a source of light.

According to these structures, the test strip (100) according to an embodiment of the present embodiment can sequentially radiate the first test light (for example, illumination light with an ultraviolet wavelength range) and the second test light (illumination light of visible light series) to the reaction area (110) thereby detecting the fluorescence and the reflected light, respectively. Therefore, in the test process regarding one target analyte (T1) which is a specific disease marker, two tests can be performed simultaneously through the fluorescence detection and the reflected light detection, and through this, the diagnosis accuracy can be improved by mutual verification effect with respect to the test result. Additionally, when the density range of a target analyte existed in the human body is high, a density in a low density range can be measured using fluorescence and a density in a high density range can be measured by reflected light, thereby improving measurement accuracy. And, a fluorescent type immunoassay analysis diagnosis apparatus or a reflected light type immunoassay analysis diagnosis apparatus can be selectively used depending on necessity.

Meanwhile, in a case illustrated in FIG. 2(b), the first reactant (P1) and the second reactant (P2) are specifically coupled to target analytes (T1, T2) different from each other in the biopsy sample, thereby forming the biosynthetic (M3) for fluorescent detection and the biosynthetic (M4) for reflected light detection, respectively.

The formed biosynthetic (M3) for fluorescent detection and the formed biosynthetic (M4) for reflected light detection are spread toward the absorption pad (105) in the capturing membrane (104) as described with respect to FIG. 2(a), during this process, as illustrated in FIG. 3(a) and FIG. 4(a), the formed biosynthetic (M3) for fluorescent detection and the formed biosynthetic (M4) for reflected light detection are captured by the biopsy material (S) applied to the reaction area (110), and, in the reaction area (110) the biosynthetic (M3) for fluorescent detection and the biosynthetic (M4) for reflected light detection are bound or coupled to the biopsy material (S), thereby forming the bio-complex (VI) for fluorescent detection and the bio-complex (V2) for reflected light detection, respectively.

As described with respect to FIG. 2(a), by forming the bio-complex (VI) for fluorescent detection and the bio-complex (V2) for reflected light detection in the reaction area (110), when the first test light (for example, illumination light with an ultraviolet wavelength range) is radiated to the reaction area (110), fluorescence is formed by the first probe material (R1) of the bio-complex (VI) for fluorescent detection and the fluorescence can be detected, and likewise, when the second test light (illumination light of visible light series) is radiated to the reaction area (110), the reflected light is formed in response to the test light by the second probe material (R2) of the bio-complex (V2) for the reflected light detection, and the reflected light can be detected.

In this case, because the target analytes (T1, T2) coupled to the first reactant (P1) and the second reactant (P2) are different from each other, the first target analyte (T1) which is two kinds of specific disease factors can be diagnosed through one time test process. Accordingly, whether the first target analyte (T1) which is a first specific disease marker exists in the biopsy sample can be diagnosed through the fluorescent detection by the radiation of the first test light, and whether the second target analyte (T1) which is a second specific disease marker exists in the biopsy sample can be diagnosed through the detection of the reflected light by the radiation of the second test light. Therefore, by simply radiating the first and second test lights sequentially, two specific disease factors can be diagnosed simultaneously through one time test process, thereby being capable of performing multiple analysis.

In sum, the test strip (100) according to an embodiment of the present disclosure is capable of performing fluorescent detection and reflected light detection related to the target analyte (T1) in the biopsy sample by using the first reactant (P1) to which fluorescent material is coupled and the second reactant (P2) to which reflected light material is coupled as reactants (PI, P2) for detection, thereby making possible to detect both fluorescence and reflected light related to the target analyte (T1) in the biopsy sample, and therefore two times test function can be performed with one time test, thereby being capable of performing mutual verification regarding the test result and increasing a range of measurable density to improve diagnosis accuracy, and specially, by being capable of performing fluorescent detection and reflected light detection for the same reaction area (110), additional reaction area (110) is not needed, thereby being possible of performing accurate and various diagnosis without increasing the length of the test strip (100).

Additionally, because a fluorescent type immunoassay analysis diagnosis apparatus or a reflected light type immunoassay analysis diagnosis apparatus is selectively applied according to the necessity of the user, the convenience of use is increased and the range of use can be more extended.

In addition, the first reactant (P1) and the second reactant (P2) are coupled to different types of target analytes (T1, T2), respectively, thereby simultaneously detecting two target analytes (T1, T2) through one time test.

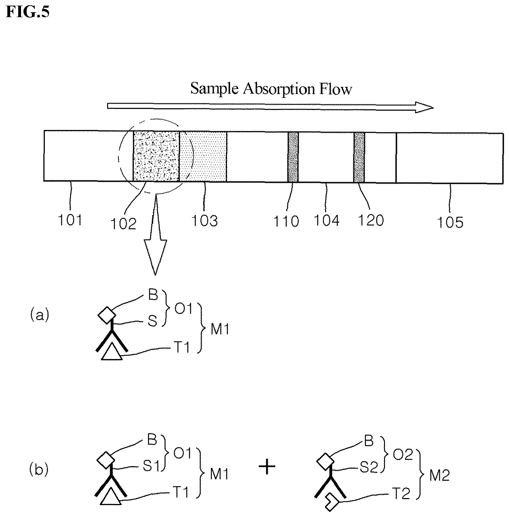

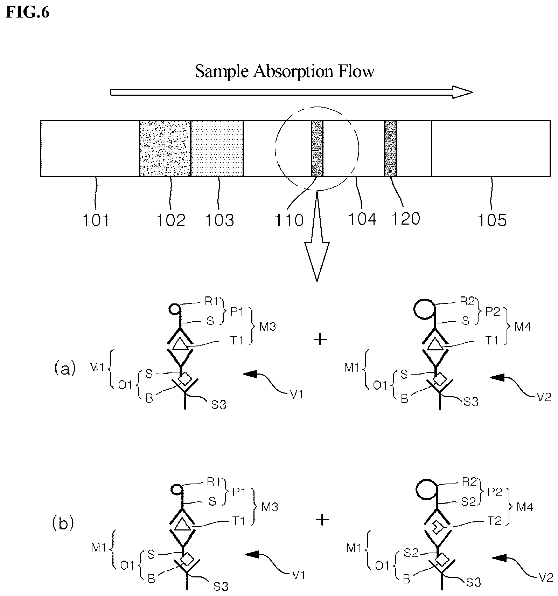

FIG. 5 is a figure of conceptually illustrating structures of reactants (O1, O2) for block of a test strip for multiplex immunoassay analysis according to an embodiment of the present disclosure, and FIG. 6 is a figure for conceptually illustrating a structure of biosynthetic captured at a reaction area in a case that a block pad (102) is included in a test strip for multiplex immunoassay analysis according to an embodiment of the present disclosure.

Referring to FIGS. 5 to 6, the reactants (O1, O2) for block is applied to the block pad (102) according to the present embodiment to be coupled to the target analytes (T1, T2) in a biopsy sample, and, in the block pad (102), the target analytes (T1, T2) and the reactants (O1, O2) for block are coupled, thereby forming biosynthetics (M1, M2) for block. In this case, in the probe pad (103), the applied reactants (P1, P2) for detection are coupled to the target analytes coupled with the reactants (O1, O2) for block.

And, the reactants (O1, O2) for block sprayed to the block pad (102) may be formed in a structure in that block material (B) is fixedly coupled to the biopsy material (S) (for example, nucleic acid comprising DNA or RNA, amino acid, fat, glycoprotein, antibody, antigen, or their combination). Additionally, material including biotin particle may be used as the block material (B).

At that time, as illustrated in FIG. 6, the capturing material (biopsy material) (S) applied to the reaction area (110) is coupled to the block material (B) of the reactant for block coupled to the target analytes (T1, T2), thereby forming bio-complexes (VI, V2). Here, if the block material (B) is biotin material, the capturing material applied to the reaction area (110) can be streptavidin. And, the capturing material (S) applied to the reaction area (110) can be material which is specifically coupled to one target analyte among the first and second target analytes (T1, T2).

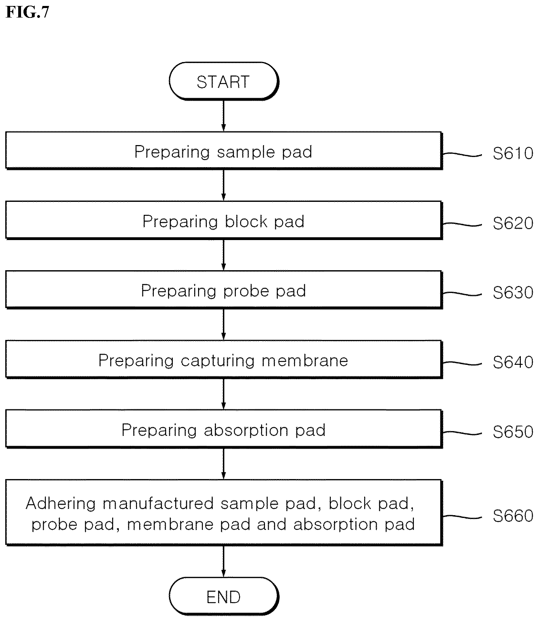

FIG. 7 is a flowchart of a process of manufacturing a test strip for multiplex immunoassay analysis according to an embodiment of the present disclosure.

Referring to FIG. 7, a process of manufacturing a test strip for multiplex immunoassay analysis according to an embodiment of the present disclosure will be described. A sample pad (101) is manufactured (S610), a block pad (102) is manufactured (S620), a probe pad (130) is manufactured (S630), a capturing membrane (104) is manufactured (S640), and an absorption pad (105) is manufactured (S650). Here, the order of manufacturing the sample pad (101), the block pad (102), the probe pad (103), the capturing membrane (104) and the absorption pad (105) can be changed, and all or some of them can be manufactured at the same time.

The manufactured sample pad (101), block pad (102), probe pad (103), capturing membrane (104) and absorption pad (105) is sequentially arranged in a direction of flow of biopsy sample, and the arranged pads can be fixed with respect to each other (S660).

At that time, the pads (101, 102, 103, 105) adhered to each other or the capturing membrane (104) are adhered such that a partial area or whole area of them can be overlapped.

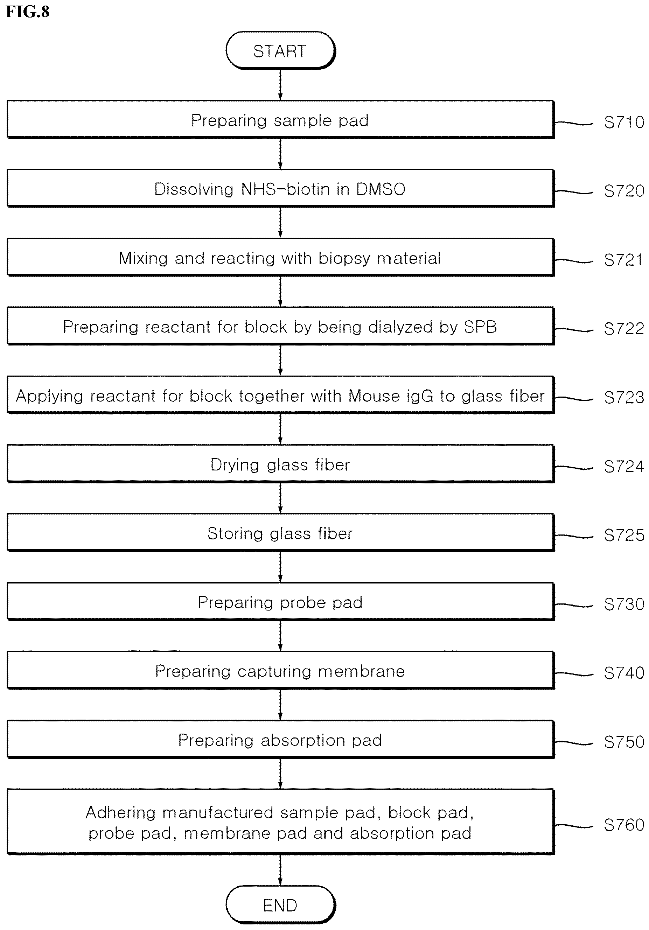

FIG. 8 is a flowchart of illustrating a process of manufacturing the block pad (102) in more detail in a process of manufacturing a test strip for multiplex immunoassay analysis according to an embodiment of the present disclosure.

Referring to FIG. 8, a process of manufacturing the block pad (102) will be described in more detail in a process of manufacturing a test strip. Firstly, the sample pad (101) is manufactured (S710).

And, NHS-biotin (N-hydroxysuccinimide-biotin) of 10 mM is dissolved in DMSO(Dimethyl sulfoxide) (S720), and the dissolved solution is mixed and reacted with the biopsy material (S) such as antibody at a room temperature for about 45 minutes (S721). The solution of which reaction is completed is dialyzed by pH 7.5 and 100 mM of SPB(sodium phosphate buffer), thereby manufacturing first and second block solutions comprising the first and second reactants (O1, O2) for block (S722). Here, both of the manufactured first and second block solutions may be C-reactive protein Biotin-antibody conjugated solution. Additionally, the manufactured first solution for block may be NT-proBNP Biotin-antibody conjugate solution (N-terminal pro b-type natriuretic peptide Biotin-antibody conjugated solution) and the second solution for block may be TnI Biotin-antibody conjugate solution (Troponin Biotin-antibody conjugated solution).

The manufactured solution for block together with mouse immunoglobulin G of 0.5 mg/mL is absorbed to a glass fiber substrate (S723). The glass fiber substrate absorbing the solution for block is dried under the pressure of 0.1 mBar or less for 1 hour (S724). Through these steps S720 to S724, the block pad (102) is manufactured and the manufactured block pad (102) is stored at the humidity of 20% or less (S725).

After that, the probe pad (103) is manufactured (S730), the capturing membrane (104) is manufactured (S740), and the absorption pad (105) is manufactured (S750). And, the manufactured sample pad (101), block pad (102), probe pad (103), capturing membrane (104) and absorption pad (105) are sequentially arranged in a flow direction of the biopsy sample, and the arranged pads can be fixed to each other (S760).

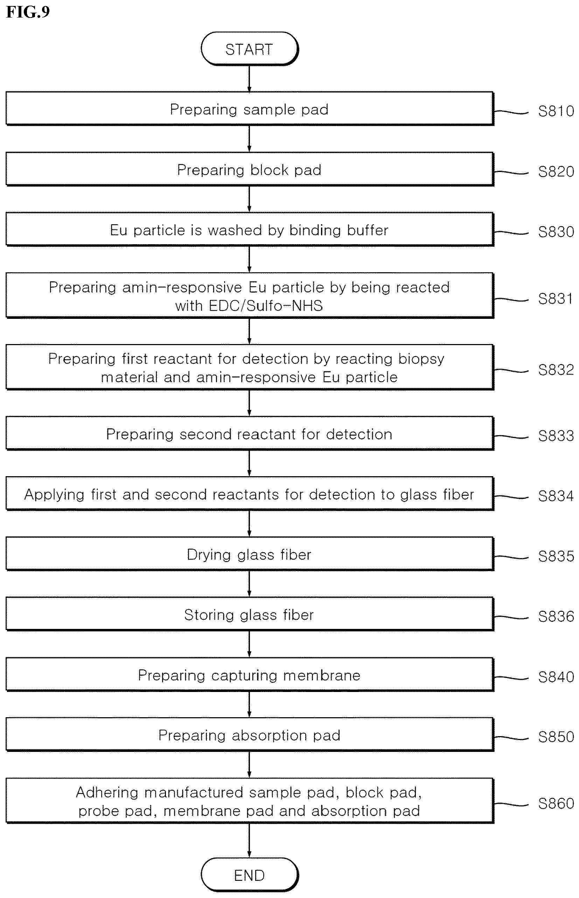

FIG. 9 is a flowchart of illustrating a process of manufacturing the probe pad (103) in more detail in a process of manufacturing a test strip for multiplex immunoassay analysis according to an embodiment of the present disclosure.

Referring to FIG. 9, a process of manufacturing the probe pad (103) will be described in more detail in a process of manufacturing a test strip. Firstly, the sample pad (101) is manufactured (S810), and the block pad (102) is manufactured (S820).

And, Eu(europium) particles having -COOH on their surfaces comprise MES(N-morpholino ethanesulfonic acid) of 50mM and is washed by a binding buffer of pH 6.1 (S830).

The washed Eu particle is reacted with EDC/Sulfo-NHS((N-(3-dimethylpropyl)-N-ethycarbodiimide hydrochloride)/N-hydroxysulfosuccinimide) at a room temperature for 30 minutes, thereby manufacturing an amin-responsive Eu particle which is a first probe material (R1) (S831).

And, the manufactured amin-responsive Eu particle is mixed and reacted with the biopsy material (S) such as antibody at a room temperature for 1 hour (S832).

By this, first probe solution having the first reactant (P1) for detection is manufactured, and here the manufactured first probe solution may be CRP Eu-antibody conjugate solution or NT-proBNP Eu-antibody conjugate solution.

When the manufacturing of the first probe solution is completed, the Eu particle is replaced with an Au particle, thereby manufacturing an amin-responsive Au particle which is second probe material, and, using this, second probe solution having the second reactant (P2) for detection is manufactured (S833). Here, the manufactured second probe solution may be CRP Au-antibody conjugate solution or TnI Au-antibody conjugate solution.

Here, the second reactant (P2) for detection may be formed by a way of ion interaction between sodium citrate ion coated on a surface of a gold particle and a cationic functional group.

If the first probe solution is CRP Eu-antibody conjugate solution and the second probe solution is CRP Au-antibody conjugate solution, the first probe solution and the second probe solution are specifically coupled to the same target analyte CRP(T1).

However, if the first probe solution is NT-proBNP Eu-antibody conjugate solution and the second probe solution is TnI Au-antibody conjugate solution, the first probe solution and the second probe solution are specifically coupled to different target analytes NT-proBNP, TnI(T1, T2), respectively.

The manufactured first probe solution and second probe solution are absorbed to a glass fiber substrate (S834), and the glass fiber substrate absorbing the first probe solution and the second probe solution is dried under the pressure of 0.1 mBar or less for 1 hour (S835). Through these steps S830 to S835, the probe pad (103) is manufactured and the manufactured probe pad

(103) is stored at the humidity of 20% or less (S836).

After that, the capturing membrane (104) is manufactured (S840), and the absorption pad

(105) is manufactured (S850). And, the manufactured sample pad (101), block pad (102), probe pad (103), capturing membrane (104) and absorption pad (105) are sequentially arranged in a flow direction of the biopsy sample, and the arranged pads can be fixed to each other (S860).

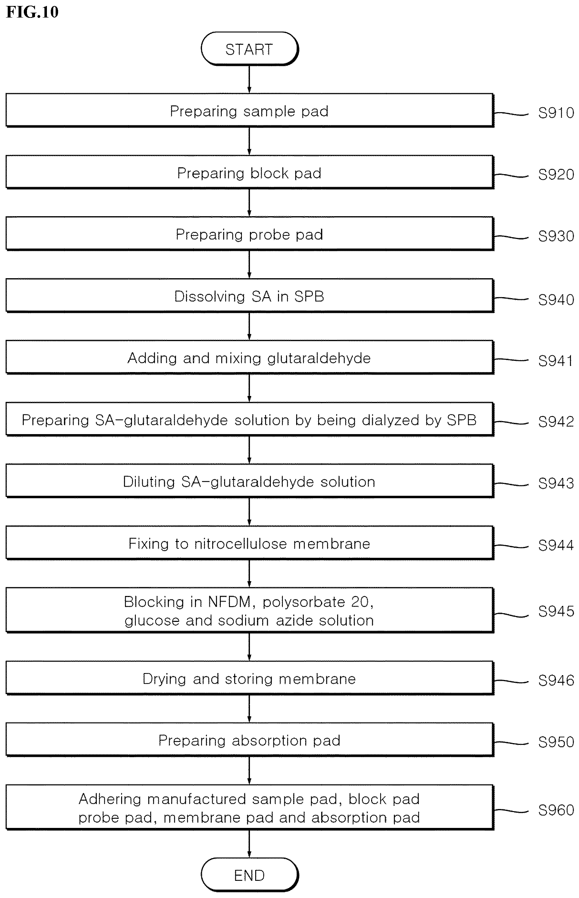

FIG. 10 is a flowchart of illustrating a process of manufacturing a capturing membrane in more detail in a process of manufacturing a test strip for multiplex immunoassay analysis according to an embodiment of the present disclosure.

Referring to FIG. 10, a process of manufacturing the capturing membrane (104) will be described in more detail in a process of manufacturing a test strip. Firstly, the sample pad (101) is manufactured (S910), the block pad (102) is manufactured (S920), and the probe pad (103) is manufactured (S930).

And, after SA(streptavidin) is dissolved in SPB of 100mM and pH 6.8 (S940), and glutaraldehyde of 1% is added to the solution, in which SA is dissolved, and then is mixed and reacted for 5 minutes (S941). The solution in which the reaction by the mixing is completed is dialyzed to SPB of 10mM and pH 6.8, thereby manufacturing SA-glutaraldehyde (streptavidin-glutaraldehyde) solution (S942).

The manufactured SA-glutaraldehyde solution is diluted to 1 mg/mL by SPB of 50mM and pH 7.5 (S943), and the diluted SA-glutaraldehyde solution is sprayed and fixed to a nitrocellulose membrane at amount of 1 uL/cm (S944).

The nitrocellulose membrane to which the SA-glutaraldehyde solution is fixed is performed of blocking for 15 minutes in a solution in which NFDM(non-fat dry milk) of 1%, polysorbate 20(tween20) of 0.05%, glucose of 1% and sodium azide of 0.005% are mixed (S945), and then is dried and stored at the humidity of 20% or less, thereby manufacturing the capturing membrane (104) (S946).

Then, after the absorption pad (105) is manufactured (S950), the manufactured sample pad (101), block pad (102), probe pad (103), capturing membrane (104) and absorption pad

(105) are sequentially arranged in a flow direction of the biopsy sample, and the arranged pads can be fixed to each other (S960).

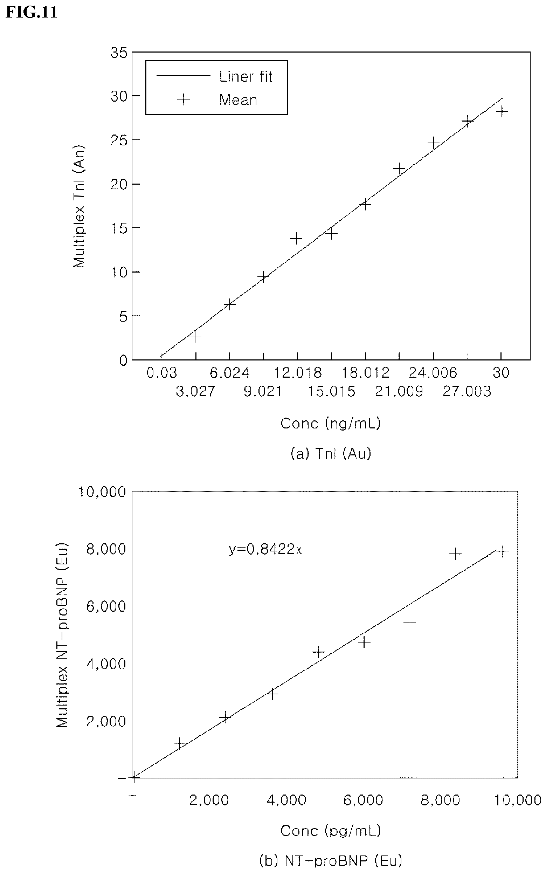

FIG. 11 is a graph showing linearity according to densities of a TnI sample and a NT-proBNP sample of a test strip (100) for multiplex immunoassay analysis according to an embodiment of the present disclosure.

Referring to FIG. 11, in order to determine the linearity according to densities of TnI sample and NT-proBNP sample of the test strip for multiplex immunoassay analysis according to an embodiment of the present disclosure, TnI samples of high density (30 ng/mL) and low density (0.03 ng/mL) and NT-proBNP samples of high density (12,000 pg/mL) and low density (30 pg/mL) were mixed with the ratios of 10:0, 9:1, 8:2, 7:3, 6:4, 5:5, 4:6, 3:7, 2:8, 1:9, 0:10, and 11 kinds of densities were made and tested. The optimal polynomial was obtained by performing polynomial regression analysis with the measured results by repeating the measurement three (3) times for each concentration of the substance. If the optimal polynomial was first order linear, it was determined that the linearity of the corresponding range was maintained, and if the optimal polynomial was not first order linear, it was determined that the linearity exists if the relative non-linearity is 15% or less.

As a result of the evaluation, the optimal polynomial was linear, TnI showed excellent linearity at 0.03 ∼ 30 ng/mL, and NT-proBNP showed excellent linearity at 30 ∼ 9,606 pg/mL. Here, the linearity was evaluated according to the instruction of CLSI EP6-A (Clinical and Laboratory Standards Institute, ed. Evaluation of the Linearity of Quantitative Measurement Procedures: a Statistical Approach; Approved Guideline. CLSI document EP6-A. Wayne, PA: Clinical and Laboratory Standards Institute, 2003.).

Therefore, it is preferable that the concentration of the TnI sample used in the preparation of the probe pad (103) according to the present embodiment is 0.03 to 30 ng/mL, and the concentration of the NT-proBNP sample is 30 to 9,606 pg/mL.

On the other hand, first, in order to set a limit of blank (LoB), the shapiro-wilk normality test was performed to a blank sample (i.e., human plasma without antigen) by repeating 60 times measurement as a process for calculating a detection limit (limit of detection, LoD) of the immunoassay test strip 100 according to the present embodiment.

When the distribution of the result values was non-normal distribution, the blank limit was set with the 95th percentile value, and in the case of normal distribution, the average and standard deviation of the measured values were obtained, and the blank limit was set according to the formula, LoB = Meanblank + 1.645SD.

The detection limit was measured 60 times each using a low concentration sample between LoB and 4 times of LoB, and because the data are normally distributed, it is calculated with the formular, LoD = LOB + cβ (cβf is the degree of freedom of SDs).

The LoB calculated by repeatedly measuring the blank sample and the low concentration sample was TnI 0.02 ng/mL and NT-proBNP 19.24 pg/mL, and the LoD was TnI 0.03 ng/mL and NT-proBNP 29.4 pg/mL.

Here, the detection limit was set according to the instruction of CLSI EP17-A2 (Clinical and Laboratory Standards Institute, ed. Evaluation of precision performance of quantitative measurement methods; approved guideline. CLSI document EP17-A2. 2nd ed. Clinical and Laboratory Standards Institute, 2012.)

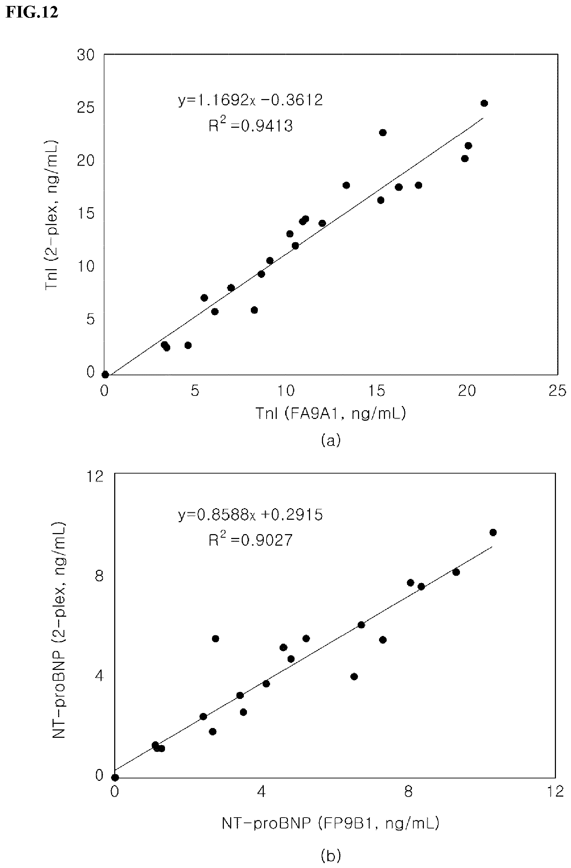

FIG. 12 is a graph of showing correlation according to concentrations of a TnI sample and a NT-proBNP sample of an immunoassay test strip according to an embodiment of the present disclosure.

Referring to FIG. 12, for each test item, Exdia TnI (LOT FA9A1, Precision Bio, KOR) and Exdia NT-proBNP (FP9B1, Precision Bio, KOR) were evaluated. A regression equation and a coefficient of correlation (r) were calculated using the values measured in each reagent. As a result of evaluating the correlation, the linear regression model was as shown in FIG. 11, and TnI followed the equation, y = 1.1692x - 0.3612, and NT-proBNP followed the equation, y = 0.8588x + 0.2915. The correlation coefficient (R 2) between two reagents was 0.9413 for TnI and 0.9027 for NT-proBNP.

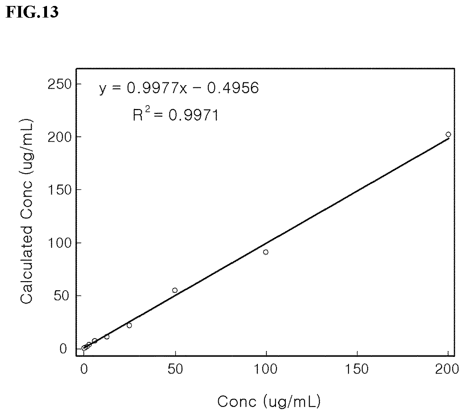

FIG. 13 is a graph of illustrating linear correlation for measuring CRP concentration of a multiple immunoassay test strip according to an embodiment of the present disclosure.

Referring to FIG. 13, in order to measure CRP of a wide concentration range in the multi-immunoassay test strip 100 according to the present embodiment, for low concentration, a signal value was measured using fluorescent particles with high sensitivity, and, for high concentration, the determination of antigen concentration was performed using a gold signal value which is easy to measure at the high concentration range but is with low sensitivity.

The process of performing determination of the concentration of the antigen will be described in more detail as follows. After reacting the antigen on the strip for 5 minutes, the intensity of the fluorescence signal value of the test line is measured. If the intensity of the fluorescence signal value is less than a predetermined cut-off, the 5pl fluorescence correction curve is applied to calculate the concentration value of the antigen, and if the measured intensity of the fluorescence signal value is greater than the cut-off, the 5pl correction curve of gold is applied to calculate the concentration value of the antigen.

In order to measure the actual sample, it is prepared by mixing the CRP antigen of 0.2-200 ug/mL with human plasma that does not contain the CRP antigen. After reacting the prepared antigen with the developed strip sensor for 5 minutes, the fluorescence signal value and the gold signal value are measured to calculate the coefficient of the 5pl calibration curve. The concentration value is calculated by applying 12.5 ug/mL, which is the section where the fluorescence signal value and the gold signal value overlap, as a cut-off. As a result of the test, the linear correlation of the sample within the range of 0.2 - 200 ug/mL followed the formula, y = 0.9977x - 0.4956, and R 2 = 0.9971 is obtained, thereby confirming that it is possible to quantitatively measure a wide range of concentrations of CRP through the present disclosure.

The foregoing descriptions have been presented in order to explain certain principles of the present disclosure by way of example, and a person having ordinary skill in the art which the present disclosure relates could make various modifications and variations without departing from the essential features of the present disclosure. Accordingly, the foregoing embodiments disclosed in the present disclosure shall be interpreted as being illustrative, while not being limitative, of the principle and scope of the present disclosure. It should be understood that the scope of the present disclosure shall be defined by the Claims and all of their equivalents fall within the scope of the present disclosure.

## Claims

1. A method of manufacturing a test strip for multiple immunoassay analysis, the method comprising:
a sample pad manufacturing step of manufacturing a sample pad so that a biopsy sample is supplied;
a probe pad manufacturing step of manufacturing a probe pad to include a first reactant for detection and a second reactant for detection coupled to target analytes, respectively;
a capturing membrane manufacturing step of manufacturing a capturing membrane to form a reaction area able to capture two biosynthetics generated by coupling the first reactant for detection and the second reactant for detection to the target analytes, respectively;
an absorption pad manufacturing step of manufacturing an absorption pad for absorption movement of the biopsy sample; and
an arrangement and fixation step of sequentially arranging and fixing the sample pad, the probe pad, the capturing membrane and the absorption pad in order of a direction of flow of the biopsy sample,
wherein:
the first reactant for detection is formed in a structure in which first probe material forming fluorescence by radiation of test light is fixedly coupled to the first reactant for detection, and
the second reactant for detection is formed in a structure in which second probe material forming reflected light by the radiation of the test light is fixedly coupled to the second reactant for detection.

2. The method of manufacturing the test strip for multiple immunoassay analysis according to claim 1, wherein the probe pad manufacturing step comprises:
a first probe solution manufacturing step of manufacturing first probe solution containing the first reactant for detection;
a second probe solution manufacturing step of manufacturing second probe solution containing the second reactant for detection;
a step in which the first probe solution and the second probe solution are absorbed to a glass fiber substrate; and
a step of drying the glass fiber substrate to which the first probe solution and the second probe solution are absorbed.

3. The method of manufacturing the test strip for multiple immunoassay analysis according to claim 2, wherein the probe pad manufacturing step further comprises a step of drying and storing the dried glass fiber substrate in a space at humidity of 20% or less.

4. The method of manufacturing the test strip for multiple immunoassay analysis according to claim 2, wherein the first probe material is an Eu (europium) particle.

5. The method of manufacturing the test strip for multiple immunoassay analysis according to claim 4, wherein the first probe solution manufacturing step comprises:
a step of washing the Eu particle having -COOH on a surface of the Eu particle by MES(N-morpholino ethanesulfonic acid) binding buffer;
a step of forming an amin-responsive Eu particle by reacting the washed Eu particle and EDC/Sulfo-NHS((N-(3-dimethylpropyl)-N-ethycarbodiimide hydrochloride)/N-hydroxysulfosuccinimide); and
a step in which the amin-responsive Eu particle is mixed and reacted with biopsy material capable of being coupled to at least one of the target analytes, and
wherein, in the step in which the amin-responsive Eu particle is mixed and reacted with the biopsy material, the amin-responsive Eu particle is coupled to the biopsy material, thereby forming the first reactant for detection.

6. The method of manufacturing the test strip for multiple immunoassay analysis according to claim 2, wherein the second probe material is an Au particle.

7. The method of manufacturing the test strip for multiple immunoassay analysis according to claim 6, wherein the second reactant for detection is formed by a way of being coupled by ion interaction between sodium citrate ion coated on a surface of a gold particle and a cationic functional group of biopsy material.

8. The method of manufacturing the test strip for multiple immunoassay analysis according to claim 1, wherein the capturing membrane manufacturing step comprises:
a step of preparing SA-glutaraldehyde (streptavidin-glutaraldehyde) solution;
a step of mixing biopsy material capable of being coupled to the target analytes with the SA-glutaraldehyde to be diluted to SPB (sodium phosphate buffer) to make diluted solution;
a step of forming the reaction area by spraying and fixing the diluted solution to a specific area of a nitrocellulose membrane; and
a step of blocking the nitrocellulose membrane processed through the step of forming the reaction area in mixed solution including NFDM (non-fat dry milk).

9. The method of manufacturing the test strip for multiple immunoassay analysis according to claim 8, wherein the capturing membrane manufacturing step further comprises a step of drying and storing the blocked nitrocellulose membrane in a space at humidity of 20% or less.

10. The method of manufacturing the test strip for multiple immunoassay analysis according to claim 8, wherein the step of preparing SA-glutaraldehyde comprises:
a step of dissolving SA (streptavidin) in the SPB;
a step of adding glutaraldehyde to solution, in which the SA is dissolved, to be mixed and reacted; and
a step of dialyzing solution, processed through the step of adding the glutaraldehyde to the solution to be mixed and reacted, with the SPB.

11. The method of manufacturing the test strip for multiple immunoassay analysis according to claim 1, further comprising a block pad manufacturing step of manufacturing a block pad to include a reactant for block coupled to the target analytes in the biopsy sample,
wherein the arrangement and fixation step sequentially arranges and fixes the sample pad, the block pad, the probe pad, the capturing membrane and the absorption pad in order of the direction of flow of the biopsy sample.

12. The method of manufacturing the test strip for multiple immunoassay analysis according to claim 11, wherein the block pad manufacturing step comprises:
a block solution preparation step of preparing block solution including the reactant for block;
a step in which the block solution together with mouse immunoglobulin G is absorbed to a glass fiber substrate; and
a step of drying the glass fiber substrate to which the block solution is absorbed.

13. The method of manufacturing the test strip for multiple immunoassay analysis according to claim 12, wherein the block pad manufacturing step further comprises a step of drying and storing the dried glass fiber substrate in a space at humidity of 20% or less.

14. The method of manufacturing the test strip for multiple immunoassay analysis according to claim 12, wherein the block pad manufacturing step further comprises:
a step of dissolving NHS-biotin (N-hydroxysuccinimide-biotin) in DMSO(Dimethyl sulfoxide);
a step of adding biopsy material capable of being coupled to at least one of the target analytes to solution, in which the NHS-biotin is dissolved, to be mixed and reacted; and
a step of dialyzing solution, processed by the step of adding the biopsy material to the solution in which the NHS-biotin is dissolved to be mixed and reacted, with the SPB,
wherein, in the step of adding the biopsy material to the solution in which the NHS-biotin is dissolved to be mixed and reacted, the NHS-biotin is coupled to the biopsy material, thereby forming the reactant for block.

15. The method of manufacturing the test strip for multiple immunoassay analysis according to claim 14, wherein, in a case that the first reactant for detection and the second reactant for detection are formed to be coupled to different types of target analytes, respectively, the reactant for block comprises a first reactant for block capable of being coupled to the first target analyte for detection, and a second reactant for block capable of being coupled to the second target analyte for detection.

16. A test strip for multiple immunoassay analysis manufactured by the method of manufacturing the test strip for multiple immunoassay analysis according to any one of claims 1 to 15.
